(19) Europäisches Patentamt
European Patent Office
Office européen des brevets

(11) EP 2 205 289 B1

(12) EUROPEAN PATENT SPECIFICATION

(45) Date of publication and mention of the grant of the patent:
**02.01.2019 Bulletin 2019/01**

(21) Application number: **08841906.4**

(22) Date of filing: **23.10.2008**

(51) Int Cl.:
***A61L 29/04*** *(2006.01)* ***A61L 29/06*** *(2006.01)*
***A61L 29/08*** *(2006.01)* ***A61L 31/04*** *(2006.01)*
***A61L 31/06*** *(2006.01)* ***A61L 31/10*** *(2006.01)*

(86) International application number:
**PCT/EP2008/064349**

(87) International publication number:
**WO 2009/053426 (30.04.2009 Gazette 2009/18)**

(54) **MEDICAL TUBINGS MADE OF A POLYMER MATERIAL**

MEDIZINISCHE SCHLÄUCHE AUS POLYMERMATERIAL

TUBULURES MÉDICALES CONSTITUÉES D'UN MATÉRIAU POLYMÈRE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **23.10.2007 US 981874 P**
**24.10.2007 US 982193 P**
**09.09.2008 US 206825**

(43) Date of publication of application:
**14.07.2010 Bulletin 2010/28**

(73) Proprietor: **Solvay Specialty Polymers USA, LLC.**
**Alpharetta, GA 30005 (US)**

(72) Inventors:
- **EL-HIBRI, Mohammad Jamal**
**Atlanta**
**Georgia 30328 (US)**
- **BALENO, Brian**
**Alpharetta**
**Georgia 30009 (US)**
- **MALJKOVIC, Nikica**
**New Orleans**
**Louisiana 70115 (US)**
- **SHEMPER, Bianca S.**
**Hattiesburg**
**Mississippi 39402 (US)**

(74) Representative: **Vande Gucht, Anne**
**Solvay S.A.**
**Département de la Propriété Industrielle**
**Rue de Ransbeek, 310**
**1120 Bruxelles (BE)**

(56) References cited:
**WO-A1-2004/004592** **WO-A1-2005/102406**
**WO-A2-2006/037078** **WO-A2-2006/094988**
**WO-A2-2007/101852** **US-A- 5 886 130**
**US-A- 6 087 467** **US-A1- 2009 082 539**

- **GRANDE J.A.: "New 'Ultra' Thermoplastic Contend for Top of Performance Pyramid" PLASTICSTECHNOLOGY, [Online] January 2007 (2007-01), pages 1-2PLASTICST, XP002557475 Retrieved from the Internet: URL:http://www.ptonline.com/articles/200701cu1.html> [retrieved on 2009-11-25]**
- **SOLVAY ADVANCED POLYMERS, L.L.C.: "Product Data PARMAX self-reinforcing polymer" INTERNET CITATION, [Online] February 2006 (2006-02), XP002557476 Retrieved from the Internet: URL:http://www.idexmedical.com/images/downloads/Parmax.pdf> [retrieved on 2009-11-25]**
- **MC MICHAEL C.: SOLVAY ADVANCED POLYMERSS, [Online] 28 July 2006 (2006-07-28), XP002556969 Retrieved from the Internet: URL:http://www.solvayadvancedpolymers.com/static/wma/pdf/9/0/3/5/Primospire%20PR250%20NT.pdf> [retrieved on 2009-11-25]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 205 289 B1

## Description

FIELD OF THE INVENTION

The present invention relates to medical tubings made of a particular polymer material.

BACKGROUND OF THE INVENTION

Medical tubings are made from a variety of materials. Glass, metal and polymers are used in a variety of medical applications. They are generally sterilized and small in diameter. Some medical tubings feature diameters that measure thousandths of an inch, with walls thinner than a human hair. These small, specialty tubes can cost many times more than conventional high-volume tubes, but are well-suited for catheters and other medical devices that are inserted into a patient's cardiovascular system. In general, medical tubing manufacturers seek to reduce the outside diameter of their tubings while maintaining as large an inside diameter as possible ; tubes with larger inside diameters provide doctors and other medical personnel with more room to insert tools or deliver drugs.

Important specifications for medical tubings include not only outside diameter and inside diameter, but also wall thickness. To produce medical tubings with extremely thin walls, manufacturers force material to flow through the small orifices of processing equipment. Gear or melt pumps are often used in the extrusion of very small tubes. Aiming at minimizing flow problems, some manufacturers use special materials for thin-wall extrusion. Examples thereof include polyether block amides, which are plasticizer-free thermoplastic elastomers that are often used in catheter tubing for angiographies, angiopplasties, endoscopies, and biopsies. The problem is that, in many instances, such special materials do not exhibit the desired balance of properties ; in particular, they provide medical tubing exhibiting a low rigidity, and additional reinforcement is then needed.

It has already been attempted to gain in rigidity by producing multilayer tubings that include reinforcements made from layers of different materials. Typically, tubings are extruded and braided over with a wire or a highly rigid polymer composition. Methods for producing multilayer tubings are by essence more complex, and further, the different layers are subject to delamination.

WO 2005/102406 (to Boston Scientific Scimed) and WO 2006/037078 (to Cordis Corp.), describe medical devices, such as catheters, made from certain rigid-rod poly(1,4-phenylene)s, as developed by Mississipi Polymer Technology under the trademark Parmax® SRP (SRP for "Self-Reinforcing Polymers"). The so-produced medical tubings are supposed to include high compression and flexural strength without reinforcing agent, as well as high chemical and wear resistance, non combustibility, high corrosion resistance, high scratch resistance, and very low moisture absorption. Unfortunately, because of the intrinsic nature of the so-proposed rigid-rod polyparaphenylene, extruding it into catheters and other medical tubings with extremely thin walls is extremely difficult ; in practice, such rigid-rod polyphenylene need to be solvent-casted into thin films from various solvent mixtures, such as NMP, with all the economic and environmental drawbacks linked to the use of solvents. Further, the torqueability and the flexibility of medical tubings made of rigid-rod poly(1,4-phenylene)s may be not as high as it would be desirable for certain applications.

Very small diameter medical tubing with very thin walls can be difficult to extrude through a standard extrusion head/die. Oftentimes, the viscosity of these materials in the die is so high and the die gap is so small that one must increase the temperature of the polymer in order to reduce the viscosity of the material so that they can get sufficient flow through the die. This practice can dramatically alter material properties. When extruding thin-walled tubing, specially designed heads are often required to produce high quality tubing without degradation, gels, black specs, or undesirable residual stress.

Many custom extruders have already tried to overcome the problems of producing tight tolerance, small diameter thin walled tubing by using high draw down ratios. This significantly improves dimensional tolerances, increases line speed and makes tooling much easier to fabricate. Unfortunately, running high draw down ratios also imparts significant orientation and residual stress/strain in the finished tubing. This orientation can significantly increase the tensile strength and reduce the elongation of the tubing in the machine direction. It can also reduce the tubing burst pressure due to the loss in hoop strength. The residual stresses from high drawn down ratios can wreak havoc during subsequent thermal processing, sterilization, or aging (natural or accelerated). These thermal processes can release the stresses built in during extrusion, causing the tubing to shrink significantly in length and increase in diameter and wall thickness.

The process used to produce medical tubing can thus be extremely important in high end diagnostic and therapeutic catheters where market pressures have driven tubing manufacturers to design smaller and smaller devices with thinner and thinner walls for end use applications where the mechanical, physical, chemical, electrical, or thermal properties are critical to the function of the finished medical tubings.

There is a need for medical tubings exhibiting a confluence of characteristics including high torqueability, high pushability and high flexibility, as well as all the above other listed benefitial properties of the rigid-rod poly(1,4-phenylene)s, and which can be easily thin-wall extruded under especially harsh conditions (e.g. by using an extruder with

extremely small orifices).

THE INVENTION

This need, and still other ones, are met by a medical tubing (T) as specified in Claim 1.

BRIEF DESCRIPTION OF THE FIGURES

The invention may be more completely understood in consideration of the following detailed description of various embodiments of the invention in connection with the accompanying drawings in which : Figure 1 depicts a diagnostic catheter or a guiding catheter.

DETAILED DESCRIPTION OF THE INVENTION

**The medical tubing (T)**

The term "tubing" is used in its broadest sense, to encompass any structure arranged at a radial distance around a longitudinal axis. Tubings, and in particular the tubings according to the present invention, are generally used for the conveyance of fluid, gas and sometimes other medium. According to the invention, the medical tubing is a catheter balloon.

Accordingly, the term "tubing" includes any structure that (i) had a hollow cylinder form with a circular inner cross-section or not, such as for example an elliptical or polygonal inner cross-section, or any other regular or irregular cross-section ; (ii) has a different or changing inner or outer cross-section along its length ; (iii) is arranged around a straight, curving, bent or discontinuous longitudinal axis ; (iv) has an imperforate surface, or a periodic or other perforate, irregular or gapped surface or cross-section ; (v) is spaced uniformly or irregularly, including being spaced varying radial distances from the longitudinal axis ; and/or (vi) has any desired combination of length or cross-sectional size.

Most specifications for medical tubing consist advantageously of a drawing of a tube with the material, dimensions and tolerances. The medical tubing according to the present invention features advantageously a single lumen. For single lumen tubings, the dimensions will usually include two of the following three dimensions ; inner diameter (ID), outer diameter (OD) and the tubing wall thickness, along with their associated tolerances. In addition, the tubing length and tolerance is typically included unless the tubing is to be provided in a continuous length on a spool. Other notes that may appear on a tubing specification include packaging requirements ; a sampling plan for inspection of the dimensional tolerances listed above ; and some note regarding tubing cleanliness such as "no dirt, grease, oil, etc. to be present on the tubing surface". Very few specifiers of medical tubing specify other tubing attributes or process parameters associated with the production of the tubing. It is a common misconception that as long as a lot of tubing is made from the right material and meets the dimensional requirements, it will be the same as, or it is equivalent to, another lot of tubing either made by the same supplier or potentially made by a different supplier. While this may be true, there is also a good chance that the two lots of tubing may be different. These differences are not always obvious or easily recognizable, even when inspected by incoming QC. Oftentimes, the process parameters and the equipment used to extrude the tubing are as important as or even more important than the actual dimensions of the tube.

The medical tubing according to the present invention can be a single lumen tubing that is characterized by its dimensions, and in particular when the lumen has a circular cross-section, the tubing inner diameter (ID), the tubing outer diameter (OD) and the tubing wall thickness (WT).

The tubing inner diameter (ID) of the medical tubing according to the present invention is advantageously below 1.9 cm. It may be below 1.6 cm, below 1.3 cm or even below 1.0 cm. The tubing inner diameter (ID) of the medical tubing according to the present invention is sometimes much smaller and may reach values of at most 0.50 cm, 0.20 cm or 0.10 cm. The tubing inner diameter (ID) of the medical tubing according to the present invention may even be below 0.09 cm.

The tubing outer diameter (OD) of the medical tubing according to the present invention is advantageously below 2.8 cm. It may be below 2.2 cm, below 1.5 cm or even below 1.0 cm. The tubing outer diameter (OD) of the medical tubing according to the present invention is sometimes much smaller and may reach values of at most 0.80 cm, 0.50 cm or 0.20 cm. The tubing outer diameter (OD) of the medical tubing according to the present invention may even be below 0.10 cm.

The tubing wall thickness (WT) of the medical tubing according to the present invention is advantageously below 0.40 cm. It may be below 0.20 cm, below 0.15 cm or even below 0.10 cm. The tubing wall thickness (WT) of the medical tubing according to the present invention is sometimes much smaller and may reach values of at most 0.080 cm, 0.050 cm or 0.020 cm. The tubing wall thickness (WT) of the medical tubing according to the present invention may even be below 0.015 cm.

For example, medical tubings according to the present invention have the following dimensions : (i) a tubing inner diameter (ID) of 0.50 cm, a tubing outer diameter (OD) of 0.90 cm and a tubing wall thickness (WT) of 0.40 cm, (ii) a tubing inner diameter (ID) of 0.35 cm, a tubing outer diameter (OD) of 0.50 cm and a tubing wall thickness (WT) of 0.150 cm, (iii) a tubing inner diameter (ID) of 0.09 cm, a tubing outer diameter (OD) of 0.11 cm and a tubing wall thickness (WT) of 0.02 cm.

When the medical tubing according to the present invention is a single lumen tubing with a non circular inner cross-section, such as a polygonal or elliptical inner cross-section, the inner diameter (ID) is calculated as the equivalent circular diameter, i.e. the diameter of the circle of equal area to that of the area of the inner cross-section. The wall thickness (WT) is then calculated as the difference between the outer diameter (OD) and the so-calculated inner diameter (ID).

The medical tubing (T) may consist of one part (the "single part"). Then, the single part consists of the polymer material (M).

Alternatively, the medical tubing (T) may consist of several parts. The case being, either one part or several parts of the medical tubing (T) may consist of the polymer material (M). When several parts of the medical tubing (T) consist of the polymer material (M), each of them may consist of the same polymer material (M) ; alternatively, at least two of them differ from each other by the chemical nature of the polymer material (M).

The medical tubing (T) in accordance with the present invention is especially useful for therapeutically or surgically treating a patient.

Examples of applications of the medical tubing (T) include high pressure catheter tubing ; tubing used to make angioplasty and stent delivery catheters ; balloon tubing used to fabricate medical balloons, especially high pressure angioplasty and stent delivery balloons ; tubing that will be implanted or inserted in the body for long periods of time ; and other

Preferred medical tubings (T) are selected from the group of catheters.

According to the invention, the medical tubing is a catheter balloon.

In a first preferred embodiment (E1), the medical tubing (T) is a medical device exactly as described in WO 2006/037078, except that the rigid-rod polyphenylene included in the medical device of WO 2006/037078 is completely replaced, weight pro weight, by the kinked rigid-rod polyphenylene (P) as above described. In particular, in accordance with embodiment (E1), the medical tubing (T) may be :

- a balloon catheter (T2) comprising : a catheter shaft having a proximal and distal end, defining an inflation lumen ; a polymer balloon affixed to the catheter shaft near the distal end, the inflation lumen communicating with an interior of the balloon ; at least a portion of the balloon catheter being made of the kinked rigid-rod polyphenylene (P) ;
- the balloon catheter (T2) as above described, wherein the balloon is made of the kinked rigid-rod polyphenylene (P) ;
- the balloon catheter (T2) as above described, wherein at least a portion of the catheter shaft is formed of the kinked rigid-rod polyphenylene (P) ;
- the balloon catheter (T2) as above described, wherein the balloon is an angioplasty balloon;
- the balloon catheter (T2) as above described, further comprising a stent crimped around the balloon, such that inflation of the balloon will expand and deploy the stent.

The self-reinforcing nature of the kinked rigid-rod polyphenylene (P) allows for thinner wall extrusions without sacrificing strength or stiffness, which translates into catheter deliverability, pushability and steerability.

Catheters of the present invention are often intended to follow a specific path through body passages selected by a physician.

Structurally, catheters may have a flexible shaft extending between a proximal end and a distal end, and may define one or more tubular passages or "lumens" extending through part or all of the catheter shaft. Such lumens often have one or more openings, referred to as "ports," or a lumen may have a closed lumen.

When a lumen is adapted to slidingly receive a guidewire, it is referred to as a "guidewire lumen," and it will generally have a proximal and distal "guidewire port". The distal guidewire port is often at or near the catheter shaft distal end.

A hub is often affixed to the catheter shaft proximal end. The hub may serve a variety of functions, including providing a handle for manipulating the catheter, and/or defining proximal port(s) communicating with lumen(s) defined by the catheter shaft. When the catheter has a guidewire lumen, a proximal guidewire port may be located at some point along the sidewall of the catheter shaft, or a hub may define the proximal guidewire port.

Catheter balloons represent this invention. Since the kinked rigid-rod polyphenylene (P) can be extruded into thin-wall tubes, biaxially orienting the tube into a high strength balloon is feasible. Since kinked rigid-rod polyphenylene boasts tensile strength of up to 30ksi, balloons prepared from this material are very thin and strong. Balloons of the kinked rigid-rod polyphenylene (P) have nominal inflation pressures well in excess of 2533125 Pascal (25 atmospheres).

Because the strength of the kinked rigid-rod polyphenylene (P) puts this property in the range of metals, and

its ability to extrude, injection mold and solvent-cast, replacement of metal components in catheter systems is possible. This feature is particularly beneficial for MR (magnetic resonance) compatible catheter systems. In the case of marker bands, kinked rigid-rod polyphenylene can be doped with appropriate radiopaque material and then extruded to create marker bands with low profile.

It is of course possible to build various kinds and designs of medical tubings according to the present invention, by various techniques and of various materials, to obtain the desired features.

Figure 1 shows for example a diagnostic catheter or a guiding catheter having a flexible tubular shaft extending between proximal and distal ends, a hub affixed to the proximal end, and a strain relief positioned at trie transition. Again, any or all of the components of the diagnostic catheter shown in Figure 1 may be made of the kinked rigid-rod polyphenylene (P).

The invention pertains to a balloon catheter such as an angioplasty or stent-delivery catheter having a balloon sleeve consisting of the polymer material (M) comprising the kinked rigid-rod polyphenylene (P). The balloon sleeve may have a first layer that is the polymer material (M) and a second layer that is another polymer material, such as a non-crosslinked nylon.

The balloon may have a wall formed using variable coextrusion, with this polymer used where certain characteristics such as non-compliance are desired and another polymer where other characteristics are desired. The balloon wall may be formed from a weave or mesh of this polymer coated with or overlaying another polymer.

Any of the medical tubings in accordance with the present invention and described herein may be provided with a coating on a surface of the tubings. Such coatings may be provided for various purposes including, but not limited to, carrying a therapeutic agent for localized delivery to a target area within the body ; providing a lubricious surface to facilitate introduction of the medical tubing into the patient during an interventional procedure ; improving the biocompatibility of the medical device with the surrounding environment; or, for a combination of such or other purposes.

The terms torqueability, pushability and flexibility are herein defined as follows. Torqueability is the ability to transmit a rotational force from a proximal portion to a distal portion. Torqueability may be advantageous if a guidewire is shaped to conform to specific vasculature, and the guidewire needs to be specifically oriented to take full advantage of its shape. Pushability is the ability to transmit a longitudinal force from a proximal portion to a distal portion so that the longitudinal displacement of the distal portion is approximately the same as the longitudinal displacement of the proximal portion. In contrast, a device that does not exhibit a high degree of pushability would displace laterally near the proximal portion, creating bends or curves in the device. Flexibility is the ability of a device to bend without breaking or permanent deformation.

Medical tubings as defined in Claim 1 are typically sterilized and small in diameter.

The tubing inner diameter (ID) of the tubing according to the present invention is advantageously below 1.9 cm. It may be below 1.6 cm, below 1.3 cm or even below 1.0 cm. The tubing inner diameter (ID) of the tubing according to the present invention is sometimes much smaller and may reach values of at most 0.50 cm, 0.20 cm or 0.10 cm. The tubing inner diameter (ID) of the tubing according to the present invention may even be below 0.09 cm.

The tubing outer diameter (OD) of the tubing according to the present invention is advantageously below 2.8 cm. It may be below 2.0 cm, below 1.5 cm or even below 1.0 cm. The tubing outer diameter (OD) of the tubing according to the present invention is sometimes much smaller and may reach values of at most 0.80 cm, 0.50 cm or 0.20 cm. The tubing outer diameter (OD) of the tubing according to the present invention may even be below 0.10 cm.

The tubing wall thickness (WT) of the tubing according to the present invention is below 0.10 cm. The tubing wall thickness (WT) of the tubing according to the present invention is sometimes much smaller and may reach values of at most 0.08 cm, 0.05 cm or 0.02 cm. The tubing wall thickness (WT) of the tubing according to the present invention may even be below 0.015 cm.

## The kinked rigid-rod polyarylene (P)

For the purpose of the present invention, an arylene group is a hydrocarbon divalent group consisting of one core composed of one benzenic ring or of a plurality of benzenic rings fused together by sharing two or more neighboring ring carbon atoms, and of two ends.

Non limitative examples of arylene groups are phenylenes, naphthylenes, anthrylenes, phenanthrylenes, tetracenylenes, triphenylylenes, pyrenylenes, and perylenylenes. The arylene groups (especially the numbering of the ring carbon atoms) were named in accordance with the recommendations of the CRC Handbook of Chemistry and Physics, 64th edition, pages C1-C44, especially p. C11-C12.

Arylene groups present usually a certain level of aromaticity ; for this reason, they are often reported as "aromatic" groups. The level of aromaticity of the arylene groups depends on the nature of the arylene group ; as thoroughly explained in Chem. Rev. 2003, 103, 3449-3605, "Aromaticity of Polycyclic Conjugated Hydrocarbons", the level of aromaticity of a polycyclic aromatic hydrocarbon can be notably quantified by the "index of benzene character" B, as defined on p. 3531 of the same paper ; values of B for a large set of polycyclic aromatic hydrocarbon are reported on

table 40, same page.

An end of an arylene group is a free electron of a carbon atom contained in a (or the) benzenic ring of the arylene group, wherein an hydrogen atom linked to said carbon atom has been removed. Each end of an arylene group is capable of forming a linkage with another chemical group. An end of an arylene group, or more precisely the linkage capable of being formed by said end, can be characterized by a direction and by a sense ; to the purpose of the present invention, the sense of the end of an arylene group is defined as going from the inside of the core of the arylene group to the outside of said core. As concerns more precisely arylene groups the ends of which have the same direction, such ends can be either of the same or opposite sense ; also, their ends can be in the straight foregoing of each other, or not (otherwise said, they can be disjoint).

A polyarylene is intended to denote a polymer of which more than 50 wt. % of the recurring units are recurring units (R) of one or more formulae consisting of an optionally substituted arylene group, provided said optionally substituted arylene group is linked by each of its two ends to two other optionally substituted arylene groups via a direct C-C linkage. That the optionally substituted arylene group is linked by each of its two ends to two other optionally substituted arylene groups via a direct C-C linkage, is an essential feature of the recurring units (R) ; thus, an arylene recurring unit which is linked by at least one of its two ends to a group other than an arylene group such as phenylene recurring units $\varphi_1$, $\varphi_2$ and $\varphi_{2'}$ below :

$$- O - \varphi_1 - S(=O)_2 - ,$$

$$- O - \varphi_2 - \varphi_{2'} - O -$$

are not recurring units (R) in the sense of the present invention.

The arylene groups of which the recurring units (R) consist can be unsubstituted. Alternatively, they can be substituted by at least one monovalent substituting group.

The monovalent substituting group is usually not polymeric in nature ; its molecular weight is preferably below 500, more preferably below 300, still more preferably below 200 and most preferably below 150.

The monovalent substituting group is advantageously a solubilizing group. A solubilizing group is one increasing the solubility of the polyarylene in at least one organic solvent, in particular in at least one of dimethylformamide, N-methylpyrrolidinone, hexamethylphosphoric triamide, benzene, tetrahydrofuran and dimethoxyethane, which can be used as solvents during the synthesis of the polyarylene by a solution polymerization process.

The monovalent substituting group is also advantageously a group which increases the fusibility of the polyarylene, i.e. it lowers its glass transition temperature and its melt viscosity, so as to desirably make the polyarylene suitable for thermoprocessing.

Preferably, the monovalent substituting group is chosen from :

- hydrocarbyls such as alkyls, aryls, alkylaryls and aralkyls ;
- halogenos such as -Cl, -Br, -F and -I;
- hydrocarbyl groups partially or completely substituted by at least one halogen atom such as halogenoalkyls, halogenoaryls, halogenoalkylaryls and halogenoaralkyls ;
- hydroxyl;
- hydrocarbyl groups substituted by at least one hydroxyl group, such as hydroxyalkyls, hydroxyaryls, hydroxyalkylaryls and hydroxyaralkyls ;
- hydrocarbyloxys [-O-R, where R is a hydrocarbyl group], such as alkoxys, aryloxys, alkylaryloxys and aralkyloxys ;
- amino ($-NH_2$) ;
- hydrocarbyl groups substituted by at least one amino group, such as aminoalkyls and aminoaryls ;
- hydrocarbylamines [-NHR or $-NR_2$, where R is a hydrocarbyl group] such as alkylamines and arylamines ;
- carboxylic acids and their metal or ammonium salts, carboxylic acid halides, carboxylic anhydrides ;
- hydrocarbyl groups substituted by at least one of carboxylic acids, metals or ammonium salts thereof, carboxylic acid halides and carboxylic anhydrides, such as -R-C(=O)OH where R is an alkyl or an aryl group ;
- hydrocarbylesters [-C(=O)OR or -O-C(=O)R, where R is a hydrocarbyl group] such as alkylesters, arylesters, alkylarylesters and aralkylesters ;
- amido [$-C(=O)NH_2$] ;
- hydrocarbyl groups substituted by at least one amido group ;
- hydrocarbylamide monoesters [-C(=O)NHR or -NH-C(=O)-R, where R is a hydrocarbyl group], such as alkylamides, arylamides, alkylarylamides and aralkylamides, and hydrocarbylamide diesters [$-C(=O)NR_2$ or $-N-C(=O)R_2$, where

R are a hydrocarbyl groups], such as dialkylamides and diarylamides ;

- sulfinic acid ($-SO_2H$), sulfonic acid ($-SO_3H$), their metal or ammonium salts,
- hydrocarbylsulfones [$-S(=O)_2-R$, where R is the hydrocarbyl group], such as alkylsulfones, arylsulfones, alkylarylsulfones, aralkylsulfones ;
- aldehyde [-C(=O)H] and haloformyls [-C(=O)X, wherein X is a halogen atom] ;
- hydrocarbylketones [-C(=O)-R, where R is a hydrocarbyl group], such as alkylketones, arylketones, alkylarylketones and aralkylketones ;
- hydrocarbyloxyhydrocarbylketones [$-C(=O)-R^1-O-R^2$, where $R^1$ is a divalent hydrocarbon group such as an alkylene, an arylene, an alkylarylene or an aralkylene, preferably a $C_1$-$C_{18}$ alkylene, a phenylene, a phenylene group substituted by at least one alkyl group, or an alkylene group substituted by at least one phenyl group ; and $R^2$ is a hydrocarbyl group, such as an alkyl, aryl, alkylaryl or aralkyl group], such as alkyloxyalkylketones, alkyloxyarylketones, alkyloxyalkylarylketones, alkyloxyaralkylketones, aryloxyalkylketones, aryloxyarylketones, aryloxyalkylarylketones and aryloxyaralkylketones ;
- any of the above groups comprising at least one hydrocarbyl group or a divalent hydrocarbon group $R^1$, wherein said hydrocarbyl group or said $R^1$ is itself substituted by at least one of the above listed monovalent substituting groups, e.g. an arylketone -C(=O)-R, where R is an aryl group substituted by one hydroxyl group ;

where :

- the hydrocarbyl groups contain preferably from 1 and 30 carbon atoms, more preferably from 1 to 12 carbon atoms and still more preferably from 1 to 6 carbon atoms ;
- the alkyl groups contain preferably from 1 to 18 carbon atoms, and more preferably from 1 to 6 carbon atoms ; very preferably, they are chosen from methyl, ethyl, n-propyl, isopropyl, n-butyl, isobutyl and tert-butyl ;
- the aryl groups are defined as monovalent groups consisting of one end and one core composed of one benzenic ring (such the phenyl group) or of a plurality of benzenic rings directly linked to each other via a carbon-carbon linkage (such as the biphenyl group) or fused together by sharing two or more neighboring ring carbon atoms (such as the naphthyl groups), and wherein the ring carbon atoms are possibly substituted by at least one nitrogen, oxygen or sulfur atom ; preferably, in the aryl groups, no ring carbon atom is substituted;
- the aryl groups contain preferably from 6 to 30 carbon atoms ; more preferably, they are phenyl groups ;
- the alkyl group which is contained in the alkylaryl groups meets the preferences of the alkyl groups as above expressed ;
- the aryl group which is contained in the aralkyl groups meets the preferences of the aryl groups as above expressed.

More preferably, the monovalent substituting group is chosen from hydrocarbylketones [-C(=O)-R, where R is a hydrocarbyl group] and hydrocarbyloxyhydrocarbylketones [$-C(=O)-R^1-O-R^2$, where $R^1$ is a divalent hydrocarbon group and $R^2$ is a hydrocarbyl group], said hydrocarbylketones and hydrocarbyloxyhydrocarbylketones being unsubstituted or substituted by at least one of the above listed monovalent substituting groups.

Still more preferably, the monovalent substituting group is chosen from arylketones and aryloxyarylketones, said arylketones and aryloxyarylketones being unsubstituted or substituted by at least one of the above listed monovalent substituting groups.

Most preferably, the monovalent substituting group is an (unsubstituted) arylketone, in particular it is phenylketone [-C(=O)-phenyl].

The core of the optionally substituted arylene group of the recurring units (R) is composed of preferably at most 3, more preferably at most 2, and still more preferably at most one benzenic ring. Then, when the core of the optionally substituted arylene group of the recurring units (R) is composed of one benzenic ring, the recurring units (R) are of one or more formulae consisting of an optionally substituted phenylene group, provided said optionally substituted phenylene group is linked by each of its two ends to two other optionally substituted arylene groups via a direct C-C linkage.

As above explained, the optionally substituted arylene group of the recurring units (R) is linked by each of its two ends to two other optionally substituted arylene groups via a direct C-C linkage. Preferably, it is linked by each of its two ends to two other optionally substituted phenylene groups via a direct C-C linkage.

As also above explained, both ends of the optionally substituted arylene group of the recurring units (R) can be characterized notably by a direction and by a sense.

A first set of recurring units (R) is composed of optionally substituted arylene groups, the ends of which

- have the same direction,
- are of opposite sense, and
- are in the straight foregoing of each other

[hereafter, rigid rod-forming arylene units (Ra)].

Non limitative examples of such optionally substituted arylene groups include :

| | |
|---|---|
| 1,4-phenylene (also named p-phenylene) | 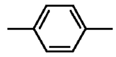 |
| 1,4-naphtylene | 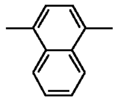 |
| 1,4-phenanthrylene and 2,7-phenanthrylene | 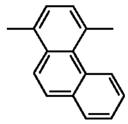 and 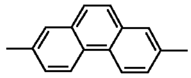 |
| 1,4-anthrylene and 9,10-anthrylene | 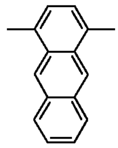 and 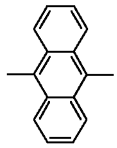 |
| 2,7-pyrenylene | 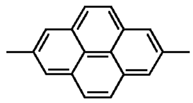 |
| 1,4-naphthacenylene and 5,12-naphthacenylene | 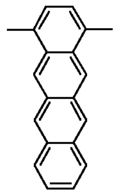 and 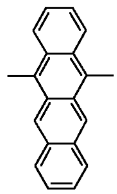 |
| 1,4-chrysenylene | 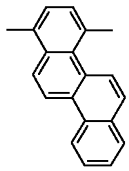 |
| 1,4-triphenylylene and 2,7-triphenylylene | 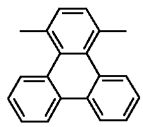 and 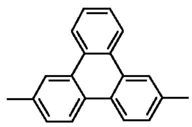 |
| 1,4-pentacenylene, 5,14-pentacenylene and 6,13-pentacenylene | 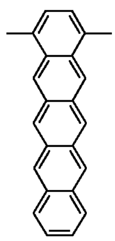 , 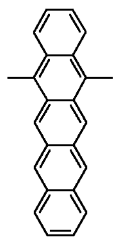 and 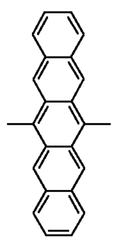 |
| 1,6-coronenylene | 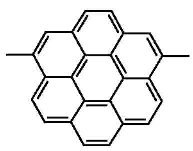 |

(continued)

| | |
|---|---|
| 1,4-trinaphthylenylene, 2,9-trinaphthylenylene and 5,18-trinaphthylenylene | 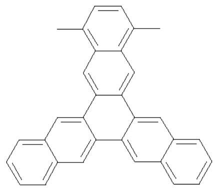 , 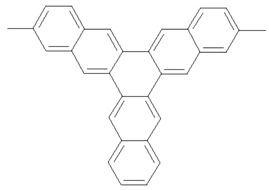 and 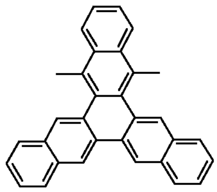 |

and any of these groups substituted by at least one monovalent substituting group, as above defined, in particular by a phenylketone group.

Optionally substituted p-phenylenes are preferred as rigid rod-forming arylene units (Ra).

Rigid rod-forming arylene units (Ra), when contained in the polyarylenes, result in straight polymer chains exhibiting an outstanding rigidity. For this reason, such polyarylenes are commonly referred to as "rigid-rod polymers".

A second set of recurring units (R) is composed of optionally substituted arylene groups, the ends of which

- either have a different direction, forming thus together an angle between 0 and 180°, said angle being possibly acute or obtuse,
- or have the same direction and the same sense,
- or have the same direction, are of opposite sense and are disjoint (i.e. not in the straight foregoing of each other)

[globally hereafter referred to as kink-forming arylene units (Rb)].

Then, a first subset of kink-forming arylene units (Rb) is composed of optionally substituted arylene groups, the ends of which have a different direction, forming together an acute angle [kink-forming arylene units (Rb-1)]. Non limitative examples of optionally substituted arylene groups the ends of which have a direction different from each other include :

| | |
|---|---|
| 1,2-phenylene (or o-phenylene) | 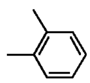 |
| 1,2-, 2,3- and 1,7-naphtylenes | 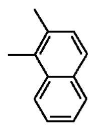 , 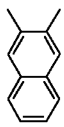 and 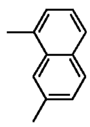 |
| 1,2-, 1,8-, 1,9-, 2,3-, 2,5- and 2,10-phenanthrylenes | 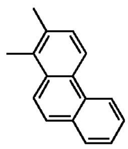 , 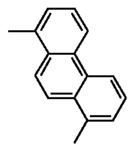 , 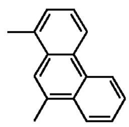 , 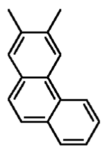 , 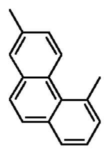 and 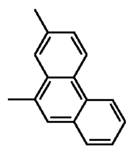 |

(continued)

| | |
|---|---|
| 1,2- and 1,7-anthrylenes | 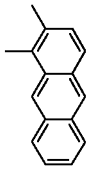 and 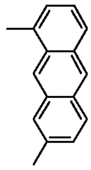 |

and any of these groups substituted by at least one monovalent substituting group, as above defined, in particular by a phenylketone group.

A second subset of kink-forming arylene units (Rb) is composed of optionally substituted arylene groups, the ends of which have a different direction, forming together an obtuse angle [kink-forming units (Rb-2)]. Non limitative examples of optionally substituted arylene groups the ends of which have a direction different from each other include :

| | |
|---|---|
| 1,3-phenylene (or m-phenylene) | 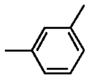 |
| 1,3 - and 1,6-naphtylenes | 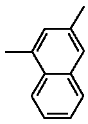 and 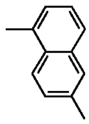 |
| 1,3-, 1,5-, 1,7-, 2,4-, 2,9- and 3,10-phenanthrylenes | 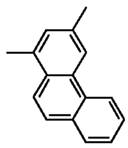, 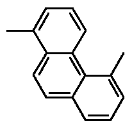, 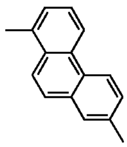, 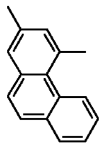, 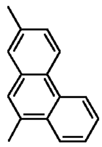 and 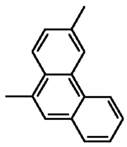 |
| 1,3- and 1,6-anthrylenes | 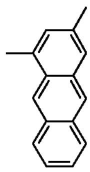 and 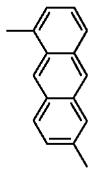 |

and any of these groups substituted by at least one monovalent substituting group, as above defined, in particular by a phenylketone group.

A third subset of kink-forming arylene units (Rb) is composed of optionally substituted arylene groups, the ends of which have the same direction and the same sense [kink-forming arylene units (Rb-3)]. Non limitative examples of optionally substituted arylene groups the ends of which the same direction and the same sense include :

| | |
|---|---|
| 1,8-naphthylene | 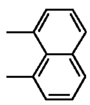 |
| 1,10- and 3,5-phenanthrylenes | 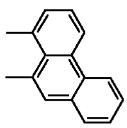 and 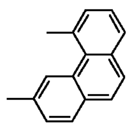 |

(continued)

| | |
|---|---|
| 1,8- and 1,9-anthrylenes | 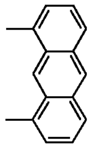 and 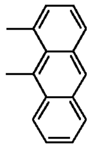 |

and any of these groups substituted by at least one monovalent substituting group, as above defined, in particular by a phenylketone group.

A fourth subset of kink-forming arylene units (Rb) is composed of optionally substituted arylene groups, the ends of which have the same direction, are of opposite sense and are disjoint [kink-forming arylene units (Rb-4)]. Non limitative examples of such optionally substituted arylene groups include :

| | |
|---|---|
| 1,5- and 2,6-naphtylenes | 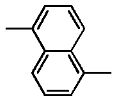 and 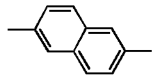 |
| 1,6-, 3,9- and 4,10-phenanthrylenes | 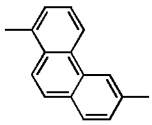, 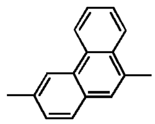 and 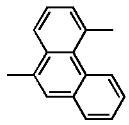 |
| 1,5-, 1,10- and 2,6-anthrylenes | 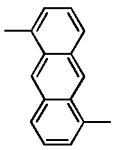, 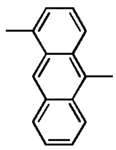 and 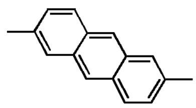 |

and any of these groups substituted by at least one monovalent substituting group, as above defined, in particular by a phenylketone group. Preferably, kink-forming arylene units (Rb) are chosen from kink-forming arylene units (Rb-1), kink-forming arylene units (Rb-2) and kink-forming arylene units (Rb-4). More preferably, kink-forming arylene units (Rb) are chosen from kink-forming arylene units (Rb-1) and kink-forming arylene units (Rb-2). Still more preferably, kink-forming arylene units (Rb) are chosen from kink-forming arylene units (Rb-1). Even still more preferably, kink-forming arylene units (Rb) are optionally substituted m-phenylenes.

Kink-forming arylene units (Rb), when contained in the polyarylene, result in more or less kinked polymer chains, exhibiting a higher solubility and fusibility than straight polymer chains. For this reason, such polyarylenes are commonly referred to as "kinked polymers".

The recurring units (R) of the kinked rigid-rod polyarylene (P) must be of a specific type, namely they must be a mix (M) consisting of :

- at least 45 mole %, and at most 55 mole %, based on the total number of moles of recurring units (R), of rigid rod-forming arylene units (Ra), said rigid rod-forming arylene units (Ra) being optionally substituted by at least one monovalent substituting group
  with
- at least 45 mole %, and at most 55 mole %, based on the total number of moles of recurring units (R), of kink-forming arylene units (Rb), said kink-forming arylene units being optionally substituted or not by at least one monovalent substituting group,

The recurring units (R) are preferably a mix (M) consisting of:

- at least 45 mole %, and at most 55 mole % based on the total number of moles of recurring units (R), of rigid rod-forming arylene units (Ra) chosen from optionally substituted p-phenylenes,
  with
- at least 45 mole %, and at most 55 mole %, based on the total number of moles of recurring units (R), of kink-forming

arylene units (Rb) chosen from (i) optionally substituted m-phenylenes and (ii) mixes of optionally substituted m-phenylenes with optionally substituted o-phenylenes.

Preferably, essentially all, if not all, the rigid rod-forming arylene units (Ra) of the mix (M) are p-phenylene units substituted by at least one substituting group. More preferably, essentially all, if not all, the rigid rod-forming arylene units (Ra) of the mix (M) are p-phenylenes substituted by at least one monovalent substituting group chosen from hydrocarbylketones [-C(=O)-R, where R is a hydrocarbyl group] and hydrocarbyloxyhydrocarbylketones [-C(=O)-$R^1$-O-$R^2$, where $R^1$ is a divalent hydrocarbon group and $R^2$ is a hydrocarbyl group], said hydrocarbylketones and hydrocarbyloxyhydrocarbylketones being themselves unsubstituted or substituted by at least one monovalent substituting group as those above listed. Still more preferably, essentially all, if not all, the rigid rod-forming arylene units (Ra) of the mix (M) are p-phenylenes substituted by at least one monovalent substituting group chosen from arylketones and aryloxyarylketones, said arylketones and aryloxyarylketones being unsubstituted or substituted by at least one monovalent substituting group as those above listed. Most preferably, essentially all, if not all, the rigid rod-forming arylene units (Ra) of the mix (M) are p-phenylenes substituted by an arylketone group, in particular by the phenylketone group.

Essentially all, if not all, the kink-forming arylene units (Rb) of the mix (M) are m-phenylene units optionally substituted by at least one substituting group. More preferably, essentially all, if not all, the kink-forming arylene units (Rb) of the mix (M) are m-phenylene units which are optionally substituted by at least one monovalent substituting group chosen from hydrocarbylketones [-C(=O)-R, where R is a hydrocarbyl group] and hydrocarbyloxyhydrocarbylketones [-C(=O)-$R^1$-O-$R^2$, where $R^1$ is a divalent hydrocarbon group and $R^2$ is a hydrocarbyl group], said hydrocarbylketones and hydrocarbyloxyhydrocarbylketones being themselves unsubstituted or substituted by at least one monovalent substituting group as those above listed. Still more preferably, essentially all, if not all, the kink-forming arylene units (Rb) of the mix (M) are unsubstituted m-phenylene units.

In the mix (M), the number of moles of the kink-forming arylene units (Rb), based on the total number of moles of the recurring units (R), is of at least 45 %. On the other hand, in the mix (M), the number of moles of the kink-forming arylene units (Rb), based on the total number of moles of the recurring units (R), is of at most 55 %.

Good results were obtained when the recurring units (R) were a mix consisting of p-phenylene units substituted by a phenylketone group with unsubstituted m-phenylene units, in a mole ratio of about 50:50.

The kinked rigid-rod polyarylene (P) may further comprise recurring units (R*), different from recurring units (R).

Recurring units (R*) may contain or not at least one strong divalent electron withdrawing group linked on each of its ends to an arylene group. Non limitative examples of recurring units (R*) free of such strong divalent electron withdrawing group are :

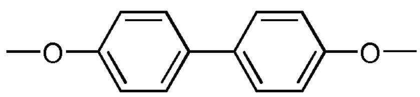

(1)

and

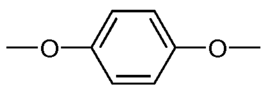

(2)

Recurring units (R*) contain preferably at least one strong divalent electron withdrawing group linked on each of its ends to an arylene group, in particular a p-phenylene group. The divalent electron withdrawing group is preferably chosen from the sulfone group [-S(=O)$_2$-], the carbonyl group [-C(=O)-], the vinylene group [-CH=CH-], the sulfoxide group [-S(=O)-], the azo group [-N=N-], saturated fluorocarbon groups like -C($CF_3$)$_2$-, organic phosphine oxide groups [-P(=O)(=$R_h$)-, where $R_h$ is a hydrocarbyl group] and the ethylidene group [-C(=$CA_2$)- , where A can be hydrogen or halogen]. More preferably, the divalent electron withdrawing group is chosen from the sulfone group and the carbonyl group. Still more preferably, recurring units (R*) are chosen from :

(i) recurring units of formula

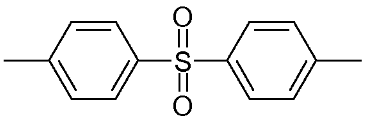

(3)

(ii) recurring units of formula

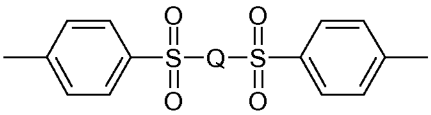

(4)

wherein Q is a group chosen from :

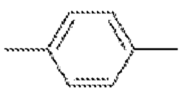 , 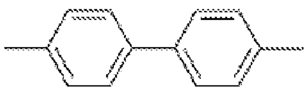 , 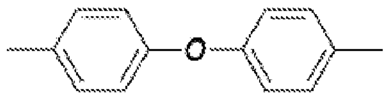 ,

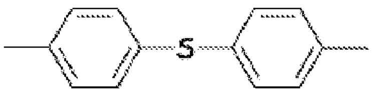 , 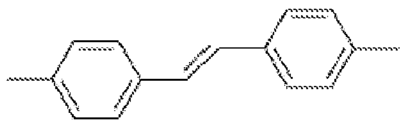 ,

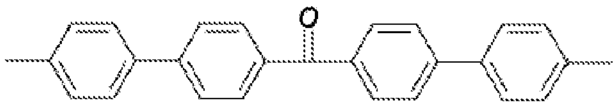 ,

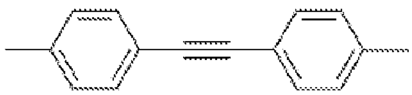 ,

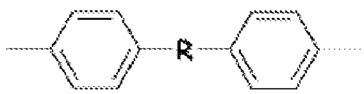

with **R** being :

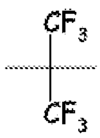 , 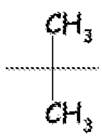 , 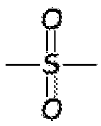 , 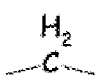 , 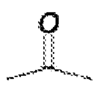 , 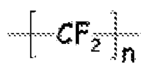 ,

$-(CH_2)_{n'}-$,
with n being an integer from 1 to 6 and n' being an integer from 2 to 6,
Q being preferably chosen from

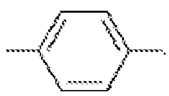 and 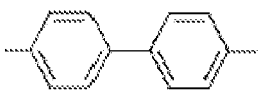 ,

(iii) recurring units of formula

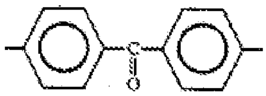

(5)

(iv) recurring units of formula

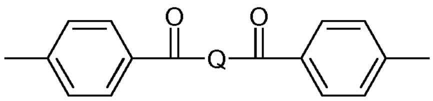

(6)

Preferably more than 75 wt. % and more preferably more than 90 wt. % of the recurring units of the polyarylene are recurring units (R). Still more preferably, essentially all, if not all, the recurring units of the polyarylene are recurring units (R).

Excellent results were obtained when the polyarylene was a kinked rigid-rod polyphenylene, essentially all, if not all, the recurring units of which consisted of a mix of p-phenylene substituted by a phenylketone group with unsubstituted m-phenylene in a mole ratio p-phenylene:m-phenylene of from 45:55 to 55:45, and most preferably of about 50:50. Such a kinked rigid-rod polyphenylene is commercially available from Solvay Advanced Polymers, L.L.C. as PRIMOSPIRE™ PR-250 polyphenylene.

The kinked rigid-rod polyarylene (P) has usually a number average molecular weight greater than 1000, preferably greater than 5000, more preferably greater than about 10000 and still more preferably greater than 15000. On the other hand, the number average molecular weight of the kinked rigid-rod polyarylene is usually below 100000, and preferably below 70000. In a certain embodiment, the number average molecular weight of the kinked rigid-rod polyarylene is above 35000. In another embodiment, it is of at most 35000 ; in this embodiment, it is often of at most 25000 and sometimes of at most 20000. The number average molecular weight of a polyarylene in general, and in particular that of the kinked rigid-rod polyarylene (P), is advantageously determined by : (1) measuring a "relative" number average molecular weight of the polyarylene by Gel Permeation Chromatography (GPC) using polystyrene calibration standards, then (2) dividing the so-measured "relative" number average molecular weight by a factor 2. It is proceeded accordingly because the skilled in the art who is a specialist of polyarylenes knows that their "relative" number average molecular weight, as measured by GPC, are generally off by a factor of about 2 times ; it has already been accounted for this correction factor in all the above cited lower and upper limits of molecular weight.

It can be amorphous (i.e. it has no melting point) or semi-crystalline (i.e. it has a melting point). It is preferably amorphous.

It has a glass transition temperature of advantageously above 50°C, preferably above 120°C and more preferably above 150°C.

The kinked rigid-rod polyarylene (P) is generally unbranched. In particular, it is generally essentially free, or even free, of recurring branching units

- *Ary* -
[ | ]x

wherein *Ary* is a polyvalent arylene and x represents the number of bonds beyond two, $x \geq 1$.

The kinked rigid-rod polyarylene (P) can be prepared by any method. A method well known in the art to prepare such kinked rigid-rod polyarylene comprises polymerizing, preferably by reductive coupling, (i) at least one dihaloarylene molecular compound consisting of an optionally substituted rigid rod-forming arylene group, which is linked on each of its two ends to one halogen atom, such as chlorine, bromine and iodine, with (ii) at least one dihaloarylene molecular compounds consisting of an optionally substituted kink-forming arylene group, which is linked on each of its two ends to one halogen atom, such as chlorine, bromine, iodine, and fluorine. The elimination of the halogen atoms from the dihaloarylene molecular compounds results in the formation of respectively optionally substituted rigid rod-forming and optionally substituted kink-forming arylene groups.

Thus, for example :

- the elimination of both chlorine atoms from a molecule of p-dichlorobenzene, p-dichlorobiphenyl or their homologous of general formula $Cl-(\varphi)_N-Cl$, N being an integer from 3 to 10, results in the formation of respectively 1, 2 or N

adjacent p-phenylene units (rigid rod-forming arylene units) ; thus, p-dichlorobenzene, p-dichlorobiphenyl and their homologous of general formula Cl-$(\varphi)_N$-Cl, N as above defined, can be polymerized, so as to form p-phenylene units ;

- 2,5-dichlorobenzophenone (p-dichlorobenzophenone) can be polymerized, so as to form 1,4-(benzoylphenylene) units (also rigid rod-forming arylene units) ;
- m-dichlorobenzene can be polymerized, so as to form m-phenylene units (kink-forming arylene units).

In the present invention, one, two, three, or even more than three different kinked rigid-rod polyarylenes (P) can be used.

**Optional ingredients**

The above described polymer material (M) may further contain one or more polymers other than the kinked rigid-rod polyarylene (P), and/or one or more non polymeric additives, collectively called optional ingredients.

The non polymeric additives of concern include notably fibrous reinforcing agents, particulate fillers and nucleating agents such as talc and silica, adhesion promoters, compatibilizers, curing agents, lubricants, metal particles, mold release agents, organic and/or inorganic pigments like $TiO_2$ and carbon black, dyes, flame retardants, smoke-suppressing agents, heat stabilizers, antioxidants, UV absorbers, tougheners such as rubbers, plasticizers, anti-static agents, melt viscosity depressants, and mixtures thereof.

In a first particular embodiment, the polymer material (M) further comprises at least one polyarylene other than the kinked rigid-rod polyarylene (P). The polyarylene other than the kinked rigid-rod polyarylene (P) is preferably a kinked rigid-rod polyarylene (P2) of which more than 50 wt. % of the recurring units are recurring units (R2) of one or more formulae consisting of an optionally substituted arylene group, provided said optionally substituted arylene group is linked by each of its two ends to two other optionally substituted arylene groups via a direct C-C linkage, said recurring units (R2) being a mix (M2) consisting of :

- from 75 mole % to 100 mole %, based on the total number of moles of the recurring units (R2), of rigid rod-forming arylene units (R2a), said rigid rod-forming arylene units (R2a) being optionally substituted by at least one monovalent substituting group,

with

- from 0 to 25 mole %, based on the total number of moles of the recurring units (R2), of kink-forming arylene units (R2b), said kink-forming arylene units (R2b) being optionally substituted by at least one monovalent substituting group.

Unless stated otherwise, the kinked-rigid rod polyarylene (P2) meets advantageously all the characteristics of the kinked-rigid rod polyarylene (P) as above detailed, at any level of preference.

The amount of the recurring units (R2a) and (R2b) of the kinked-rigid rod polyarylene (P2), the number of moles of the kink-forming arylene units (R2b) in the mix (M2), based on the total number of moles of the recurring units (R2), is preferably of at least 1.0 %, more preferably at least 5 % and still more preferably at least 10 %. On the other hand, in the mix (M2), the number of moles of the kink-forming arylene units (R2b), based on the total number of moles of the recurring units (R2), is preferably of at most 20 %, and more preferably of at most 18 %. Good results are obtained when the polyarylene (P2) is a kinked rigid-rod polyphenylene copolymer, essentially all, if not all, the recurring units of which consist of a mix (M2) of p-phenylene substituted by a phenylketone group with unsubstituted m-phenylene in a mole ratio p-phenylene:m-phenylene of from 80:20 to 95:5, preferably of from 80:20 to 90:10, and still more preferably of about 85:15. Such a kinked rigid-rod polyphenylene copolymer is commercially available from Solvay Advanced Polymers, L.L.C. as PRIMOSPIRE™ PR-120 polyphenylene.

In this first particular embodiment, the weight of the polyarylene (P2), based on the total weight of the polymer material (M), may be of at least 1 %, at least 5 %, of at least 10 %, or at least 15 % ; on the other hand, the weight of the polyarylene (P2), based on the total weight of the material, may be of of at most 99 %, of at most 95 %, of at most 75 %, or of at most 60 %.

In another particular embodiment, the polymer material (M) further comprises at least one thermoplastic polymer other than a polyarylene, selected from the group consisting of polyamides, polyether block amides, polyimides, polyetherimides, polyamideimides, polyarylethersulfones (such as polyphenylsulfones, bisphenol A polysulfones, polyethersulfones, polyetherethersulfones, polyethersulfoneimides and copolymers and mixtures thereof), polyetherketones, polyetheretherketones, polyetherketoneketones, polyarylene ethers [such as polyphenylene ethers and poly(2,6-dimethy-1,4-phenylene ether)s], polyphenylene sulfides, polybenzimidazoles, polycarbonates, polyesters, polyurethanes, polyolefins, poly(methyl pentene)s, polytetrafluoroethylenes, polyethylenes, polypropylenes, liquid crystalline polymers, halogenated polymers, and copolymers and mixtures thereof.

In still another particular embodiment, the polymer material (M) further contains at least one fibrous reinforcing agent, in particular an inorganic fibrous reinforcing agent such as glass fiber or carbon fiber, usually in an amount of from 10 to 50 wt. %, based on the total weight of the polymer material (M).

The weight of the optional ingredients, based on the total weight of the material, ranges advantageously from 0 to 75 wt. %, preferably from 0 to 50 wt. %, more preferably from 0 to 25 wt. % and still more preferably from 0 to 10 wt. %, based on the total weight of the polymer material (M). Excellent results are obtained when the material is essentially free, or is even completely free, of said optional ingredients.

## Claims

**1.** A medical tubing (T) having a wall thickness (WT) of below 0.10 cm, comprising at least one part consisting of a polymer material (M) comprising
at least one kinked rigid-rod polyarylene (P) of which more than 50 wt. % of the recurring units are recurring units (R) of one or more formulae consisting of an optionally substituted arylene group, provided said optionally substituted arylene group is linked by each of its two ends to two other optionally substituted arylene groups via a direct C-C linkage, said recurring units (R) being a mix (M) consisting of :

- at least 45 mole %, and at most 55 mole % based on the total number of moles of the recurring units (R), of rigid rod-forming arylene units (Ra), said rigid rod-forming arylene units (Ra) being optionally substituted by at least one monovalent substituting group,

with

- at least 45 mole %, and at most 55 mole % based on the total number of moles of the recurring units (R), of kink-forming arylene units (Rb), said kink-forming arylene units (Rb) being optionally substituted by at least one monovalent substituting group,

said medical tubing (T) being a catheter balloon possessing an inflation pressure well in excess of 2533125 Pascal (25 atmospheres).

**2.** The medical tubing according to claim 1, wherein the recurring units (R) of the kinked rigid-rod polyarylene (P) are a mix (M) consisting of:

- at least 45 mole %, and at most 55 mole %, based on the total number of moles of recurring units (R), of rigid rod-forming arylene units (Ra) chosen from p-phenylenes optionally substituted by at least one monovalent substituting group,

with

- at least 45 mole %, and at most 55 mole %, based on the total number of moles of recurring units (R), of kink-forming arylene units (Rb) chosen from (i) m-phenylenes optionally substituted by at least one monovalent substituting group and (ii) mixes of m-phenylenes with o-phenylenes, wherein both m-phenylenes and o-phenylenes are, independently from each other, optionally substituted by at least one monovalent substituting group.

**3.** The medical tubing according to claim 1 or 2, wherein the rigid rod-forming arylene units (Ra) of the mix (M) are p-phenylenes substituted by a phenylketone group and the kink-forming arylene units (Rb) of the mix (M) are unsubstituted m-phenylenes.

**4.** The medical tubing according to any one of the preceding claims, wherein the kinked rigid-rod polyarylene (P) is a kinked rigid-rod polyphenylene, essentially all the recurring units of which consist of a mix of p-phenylene substituted by a phenylketone group with unsubstituted m-phenylene in a mole ratio p-phenylene:m-phenylene of from 45:55 to 55:45.

**5.** The medical tubing according to any one of the preceding claims, wherein the tubing wall thickness (WT) is below 0.05 cm.

**Patentansprüche**

1. Medizinischer Schlauch (T) mit einer Wanddicke (WT) von unter 0,10 cm, umfassend wenigstens einen Teil, der aus einem Polymermaterial (M) besteht, das wenigstens ein geknicktes-starres-Stäbchen-Polyarylen (P) umfasst, bei dem mehr als 50 Gew.-% der Wiederholungseinheiten Wiederholungseinheiten (R) mit einer oder mehreren Formeln sind, die aus einer gegebenenfalls substituierten Arylengruppe bestehen, vorausgesetzt, dass die gegebenenfalls substituierte Arylengruppe mit jedem ihrer beiden Enden über eine direkte C-C-Verknüpfung an zwei andere gegebenenfalls substituierte Arylengruppen gebunden ist, wobei die Wiederholungseinheiten (R) ein Gemisch (M) sind, bestehend aus :

    - wenigstens 45 mol-% und höchstens 55 mol-%, bezogen auf die Gesamtmolzahl der Wiederholungseinheiten (R), starre-Stäbchen-bildende Aryleneinheiten (Ra), wobei die starre-Stäbchen-bildenden Aryleneinheiten (Ra) gegebenenfalls mit wenigstens einer einwertigen Substituentengruppe substituiert sind,

    mit

    - wenigstens 45 mol-% und höchstens 55 mol-%, bezogen auf die Gesamtmolzahl der Wiederholungseinheiten (R), knickbildende Aryleneinheiten (Rb), wobei die knickbildenden Aryleneinheiten (Rb) gegebenenfalls mit wenigstens einer einwertigen Substituentengruppe substituiert sind,

    wobei der medizinische Schlauch (T) ein Katheterballon ist, der einen Aufblähdruck von deutlich über 2533125 Pascal (25 Atmosphären) aufweist.

2. Medizinischer Schlauch gemäß Anspruch 1, wobei die Wiederholungseinheiten (R) des geknicktes-starres-Stäbchen-Polyarylens (P) ein Gemisch sind, bestehend aus :

    - wenigstens 45 mol-% und höchstens 55 mol-%, bezogen auf die Gesamtmolzahl der Wiederholungseinheiten (R), starre-Stäbchen-bildende Aryleneinheiten (Ra) ausgewählt aus p-Phenylenen, die gegebenenfalls mit wenigstens einer einwertigen Substituentengruppe substituiert sind,

    mit

    - wenigstens 45 mol-% und höchstens 55 mol-%, bezogen auf die Gesamtmolzahl der Wiederholungseinheiten (R), knickbildende Aryleneinheiten (Rb) ausgewählt aus (i) m-Phenylenen, die gegebenenfalls mit wenigstens einer einwertigen Substituentengruppe substituiert sind, und (ii) Gemischen von m-Phenylenen mit o-Phenylenen, wobei sowohl die m-Phenylene als auch die o-Phenylene unabhängig voneinander gegebenenfalls mit wenigstens einer einwertigen Substituentengruppe substituiert sind.

3. Medizinischer Schlauch gemäß Anspruch 1 oder 2, wobei die starre-Stäbchen-bildenden Aryleneinheiten (Ra) des Gemischs (M) p-Phenylene sind, die mit einer Phenylketongruppe substituiert sind, und die knickbildenden Aryleneinheiten (Rb) des Gemischs (M) unsubstituierte m-Phenylene sind.

4. Medizinischer Schlauch gemäß einem der vorstehenden Ansprüche, wobei das geknicktes-starres-Stäbchen-Polyarylen (P) ein geknicktes-starres-Stäbchen-Polyphenylen ist, bei dem im Wesentlichen alle Wiederholungseinheiten aus einem Gemisch von p-Phenylen, das mit einer Phenylketongruppe substituiert ist, mit unsubstituiertem m-Phenylen in einem Molverhältnis von p-Phenylen:m-Phenylen von 45:55 bis 55:45 bestehen.

5. Medizinischer Schlauch gemäß einem der vorstehenden Ansprüche, wobei die Wanddicke des Schlauchs (WT) kleiner als 0,05 cm ist.

**Revendications**

1. Tubulure médicale (T) ayant une épaisseur de paroi (WT) inférieure à 0,10 cm, comprenant au moins une partie constituée d'un matériau de polymère (M) comprenant

    - au moins un polyarylène à tige rigide torsadée (P) dont plus de 50 % en poids des motifs récurrents sont des motifs récurrents (R) d'une ou plusieurs formules constituées d'un groupe arylène facultativement substitué, à

condition que ledit groupe arylène facultativement substitué soit lié par chacune de ses deux extrémités à deux autre groupes arylène facultativement substitués par l'intermédiaire d'une liaison C-C directe, lesdits motifs récurrents (R) étant un mélange (M) constitué de :

- au moins 45 % en moles, et au plus 55 % en moles sur la base du nombre total de moles des motifs récurrents (R), de motifs d'arylène formant une tige rigide (Ra), lesdits motifs d'arylène formant une tige rigide (Ra) étant facultativement substitués par au moins un groupe de substitution monovalent,

avec

- au moins 45 % en moles, et au plus 55 % en moles sur la base du nombre total de moles des motifs récurrents (R), de motifs d'arylène formant une torsion (Rb), lesdits motifs d'arylène formant une torsion (Rb) étant facultativement substitués par au moins un groupe de substitution monovalent,

ladite tubulure médicale (T) étant un ballonnet de cathéter comportant un puits de pression de gonflage supérieur à 2533125 Pascal (25 atmosphères).

2. Tubulure médicale selon la revendication 1, dans laquelle les motifs récurrents (R) du polyarylène à tige rigide torsadée (P) sont un mélange (M) constitué de :

- au moins 45 % en moles, et au plus 55 % en moles, sur la base du nombre total de moles de motifs récurrents (R), de motifs d'arylène formant une tige rigide (Ra) choisis parmi des p-phénylènes facultativement substitués par au moins un groupe de substitution monovalent,

avec

- au moins 45 % en moles, et au plus 55 % en moles, sur la base du nombre total de moles de motifs récurrents (R), de motifs d'arylène formant une torsion (Rb) choisis parmi (i) des m-phénylènes facultativement substitués par au moins un groupe de substitution monovalent et (ii) des mélanges de m-phénylènes avec des o-phénylènes, dans lesquels les m-phénylènes et les o-phénylènes sont, indépendamment les uns des autres, facultativement substitués par au moins un groupe de substitution monovalent.

3. Tubulure médicale selon la revendication 1 ou 2, dans laquelle les motifs d'arylène formant une tige rigide (Ra) du mélange (M) sont des p-phénylènes substitués par un groupe phénylcétone et les motifs d'arylène formant une torsion (Rb) du mélange (M) sont des m-phénylènes non substitués.

4. Tubulure médicale selon l'une quelconque des revendications précédentes, dans laquelle le polyarylène à tige rigide torsadée (P) est un polyphénylène à tige rigide torsadée, dont pratiquement tous les motifs récurrents sont constitués d'un mélange de p-phénylène substitué par un groupe phénylcétone avec un m-phénylène non substitué dans un rapport molaire p-phénylène:m-phénylène de 45:55 à 55:45.

5. Tubulure médicale selon l'une quelconque des revendications précédentes, dans laquelle l'épaisseur de paroi de tubulure (WT) est inférieure à 0,05 cm.

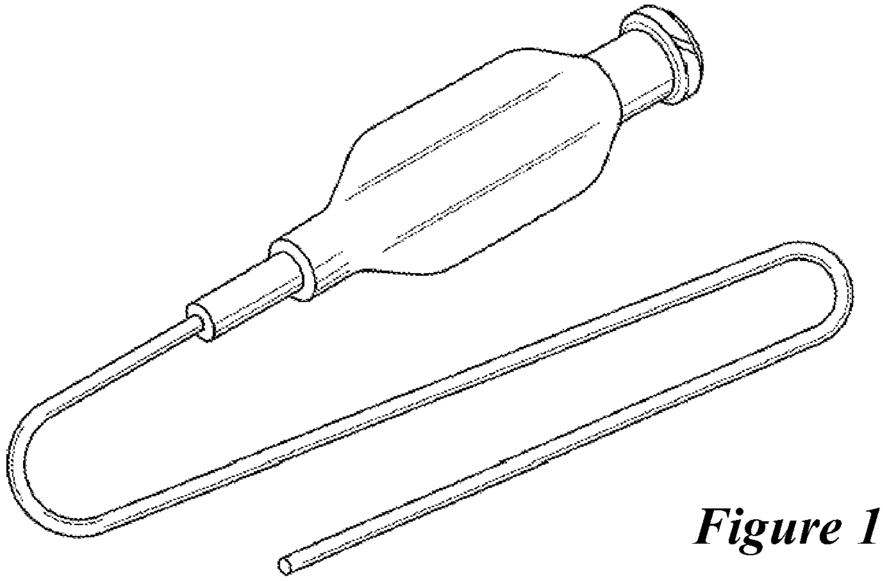

*Figure 1*

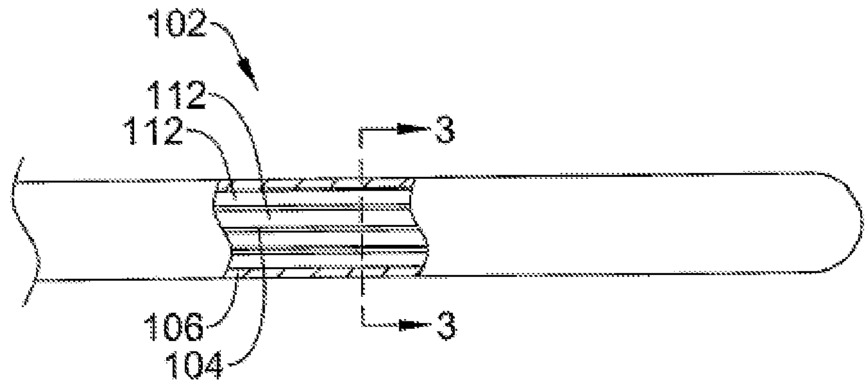


*Figure 2*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005102406 A
- WO 2006037078 A

### Non-patent literature cited in the description

- CRC Handbook of Chemistry and Physics. C1-C44
- Aromaticity of Polycyclic Conjugated Hydrocarbons. *Chem. Rev.,* 2003, vol. 103, 3449-3605